# EUROPEAN PATENT SPECIFICATION

(11) **EP 1 133 262 B1**
(45) Date of publication and mention of the grant of the patent: **18.09.2002**
(21) Application number: 99954206.1
(22) Date of filing: 10.11.1999
(51) Int. Cl.: A61B 17/064

(54) **A SURGICAL DEVICE FOR CLOSING TISSUE**
CHIRURGISCHES GEWEBEBINDEELEMENT
DISPOSITIF CHIRURGICAL D'OBTURATION DE TISSUS

(30) Priority: 25.11.1998 GB 9825681
(43) Date of publication of application: 19.09.2001
(73) Proprietor: Biocomposites Limited, Stoke-on-Trent, Staffordshire ST1 5PQ (GB)
(72) Inventor: BRATT, John Stephen Biocomposites Limited, Stoke-on-Trent Staffordshire ST1 5PQ (GB); WATERS, Russell David Biocomposites Limited, Stoke-on-Trent Staffordshire ST1 5PQ (GB); COOPER, John Joseph Biocomposites Limited, Stoke-on-Trent Staffordshire ST1 5PQ (GB)
(74) Representative: Swindell & Pearson
(86) International application number: GB9903744
(87) International publication number: WO00030552

(56) References cited:
- US-A- 5 013 316
- US-A- 5 562 704
- US-A- 5 827 298

## Description

This invention concerns a surgical device, and particularly but not exclusively a surgical device for closing torn or incised soft bodily tissue.

A number of arrangements have been devised for closing tears, wounds and incisions in soft bodily tissue, and in particular for repairing torn menisci. These devices include sutures, clamps, clips, pins and staples, which have met with varying success. In such situations it is desired to cause as little trauma as possible whilst providing a good repair to the tissue and preferably not requiring further surgery to remove the device to reduce the risk of long term infection from non-absorbable devices.

US-A-5 013 316 discloses a surgical device comprising a shaft, a hollow cap at the proximal end of the shaft, and a plurality of tissue engagement formations on the shaft.

According to the present invention there is provided a surgical device as defined in claim 1.

The distal end of the shaft may be provided with a sharp tip, which may be enlarged relative to the shaft. Alternatively the distal end of the shaft may be blunt, and may be rounded.

The cap is preferably arranged such that in use it contacts the surface of the tissue substantially circumferentially even when the shaft is inserted other than normally to the tissue surface.

The cap may have a substantially frusto conical shape with the shaft being attached to the proximal smaller end thereof. One or more openings may be provided extending partway up the side walls of the cap from the distal larger end thereof.

The engagement formations are preferably provided at a 120° or less spacing around the shaft. The formations may be provided in a plurality of groups of formations, with each group spaced longitudinally from each other on the shaft. The formations are preferably circumferentially equispaced. The formations may be in the form of barbs, which are preferably rearwardly pointing. Engagement formations may be provided on or adjacent the distal end of the shaft.

The shaft preferably has a uniform cross-section along its length from the distal end to the cap. The shaft cross-section may be circular, elliptical or polygonal, and especially triangular or square. Alternatively the cross-section may have a plurality of circumferential lobes, which lobes are desirably equispaced.

The cap preferably comprises a formation engageable with a surgical tool, and the formation is preferably provided on the proximal end of the cap, and may comprise a recess or projection. The formation is preferably such as to permit the device to be used arthroscopically.

The device is preferably made of a resorbable material, and desirably includes a resorbable polymer or copolymer. The polymer or copolymer may be from the range of bioabsorbable polymers including β-hydroxybutyrate (hydroxybutanoate), hydroxyvalerate (hydroxypentanoate), lactic acid (2-hydroxypropanoic acid) or glycolic acid (2-hydroxyethanoic acid).

The resorbable material is preferably a composite and also comprises a particulate or fibrous solid material. The solid material may comprise any of hydroxyapatite, tri-calcium phosphate, calcium carbonate, calcium sulphate, magnesium oxide or bioactive glass

Embodiments of the present invention will now be described by way of example and with reference to the accompanying drawings, in which:-
Fig. 1 is a diagrammatic isometric side view of a device according to the invention;
Figs. 2-5 are diagrammatic cross-sectional side views of a top part of the device of Fig. 1 in use; and
Figs. 6-12 show diagrammatic side views of further devices according to the invention.

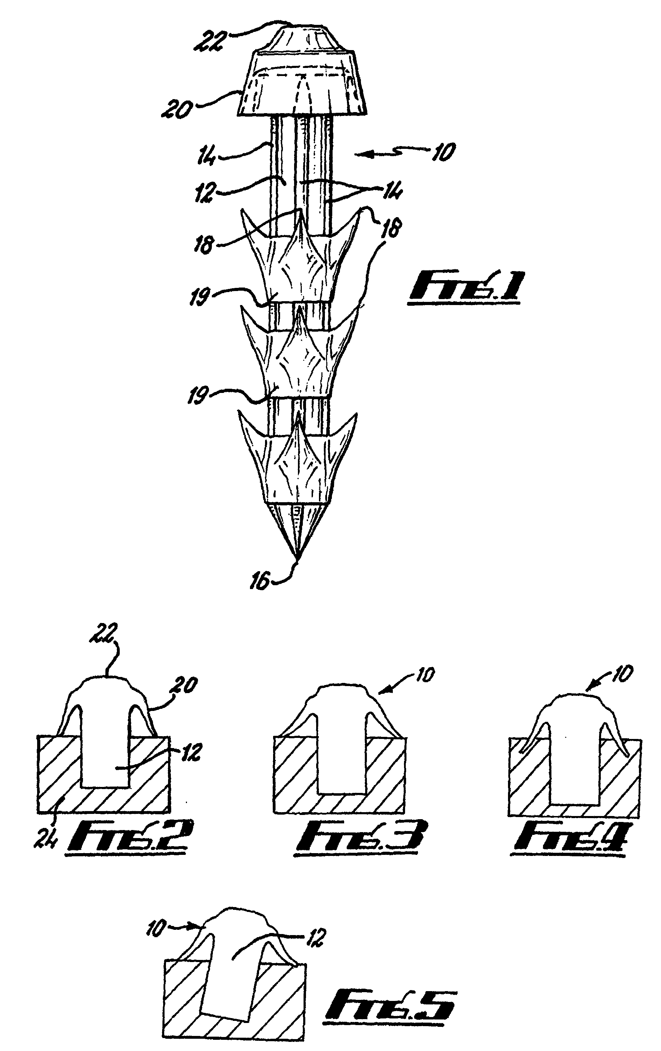

Figs. 1-5 show a surgical device 10 suitable for use for closing tears, wounds and incisions in bodily tissue, and is particularly suited to the repair of torn menisci in the knee. The device 10 comprises a shaft 12 which has a basic circular cross-section with four equispaced circumferential lobes 14 extending therefrom. The shaft 10 is provided with a sharp point 16 at its distal end.

A plurality of tissue engagement formations are provided on the shaft 12 in the form of barbs 18. The barbs 18 are provided on sleeves 19 which intimately fit on the shaft 12, and the barbs 18 are provided on the lobes 14.

A cap 20 is provided on the proximal end of the shaft 12 and the cap has a hollow frusto conical configuration pointing away from the distal end, with a dome 22 of a smaller diameter provided on the proximal end thereof. The dome 22 is engageable with a pusher or other tool to permit the device to be arthroscopically inserted.

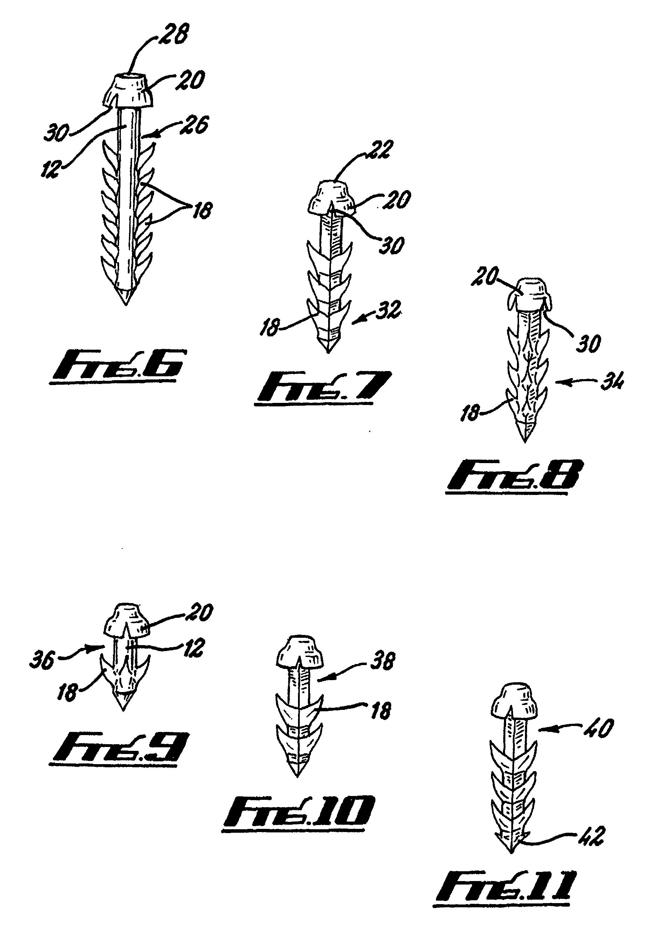

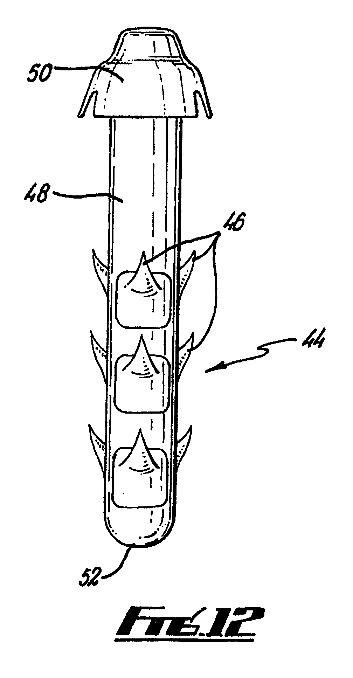

Figs. 2-4 shows an upper part of the device 10 being inserted into bodily tissue 24. In Fig. 2 the bottom edge of the cap 20 is just engaging against the top surface of the tissue 24. In Fig. 3 the device 10 has been pushed further in causing the bottom edge of the cap 20 to deform outwardly. In Fig. 4 due to the softness of the tissue 24 the bottom edge of the cap 20 has entered the tissue but due to the construction of the head can only enter a relatively short distance. Fig. 5 illustrates the condition where the shaft 12 has been inserted into the tissue 24 at an inclined angle. In this instance the cap 20 deforms to provide a circumferential engagement with the tissue 24.

The device 10 therefore provides a good circumferential engagement with the surface of the tissue or engaging slightly into the tissue irrespective of whether the shaft is normally inserted thereinto. If movement of the tissue occurs as a result of movement of the body, this can be accommodated by the cap so as to maintain circumferential engagement throughout the movement yet permitting movement to occur.

The device 10 is made of polylactide which will resorb within the body thus eliminating the need for further surgery to remove the device and also preventing the risk of long term infection which a non-absorbable device would present. Other bioabsorbable polymers such as polyglycolide or polydioxanone are suitable materials for this device.

Figs. 6-12 show further devices according to the invention. In Fig. 6 a device 26 is shown. The shaft 12 of the device 26 has an ellipsoidal cross-section, and two sets of six barbs 18 are provided on opposite sides of the shaft 12. The cap 20 is a similar shape to that provided on the device 10 except that a recess 28 is provided instead of a dome to engage with a pusher. A split 30 is provided extending partway up the edge of the cap 20 to permit further flexing of the cap.

Fig. 7 shows a device 32 of triangular cross-section with three sets of three barbs 18 mounted on each of the faces of the triangular cross-section. The cap 20 is provided with a dome 22 and also a split 30. Fig. 8 shows a further device 34 which is similar to the device 32 except that the barbs 18 are provided on the apices of the cross-section, and three equispaced splits 30 are provided in the cap 20.

Fig. 9 shows a smaller device 36 of circular cross-section with four barbs 18 equispaced around the shaft 12 adjacent the distal end thereof. The cap 20 of the device 36 is similar to that of the device 32. Fig. 10 shows a device 38 of square cross-section with two barbs 18 on each face of the shaft 12. Fig. 11 shows a further device 40 which is similar to the device 32 except that an enlarged distal head 42 is provided.

Fig. 12 shows a still further surgical device 44. The device 44 has a similar configuration of barbs 46 to the device 10, but each barb 46 is mounted separately on the shaft 48. The cap 50 is similar to the cap 20. The distal end 52 of the shaft 48 is in contrast rounded. The rounded end 52 means that the device 44 is not self-penetrating and therefore a tract would initially require to be made in the tissue. A rounded end has the advantage of causing less tissue irritation than usually occurs with a pointed end.

Various other modifications or combinations of the above features can be included. For instance different cross-sections of the shaft could be provided and different shape barbs could also be provided. A different shaped cap and perhaps with a different number or size of splits therein could be used.

## Claims

1. A surgical device for closing bodily tissue, the device comprising a shaft (12, 48) which can be inserted into the tissue, a hollow cap (20, 50) at the proximal end of the shaft (12, 48) which is engageable substantially against the surface of the tissue, and a plurality of tissue engagement formations (18,46) on the shaft (12, 48) to prevent the device from being withdrawn from the tissue, **characterised in that** the cap (20, 50) is flexible so that a good circumferential engagement with the surface of the tissue is provided.

2. A surgical device according to claim 1, **characterised in that** the distal end of the shaft (12, 48) is provided with a sharp tip (16).

3. A surgical device according to claim 1, **characterised in that** the distal end (52) of the shaft (12, 48) is blunt.

4. A surgical device according to claim 3, **characterised in that** the distal end (52) of the shaft (12, 48) is rounded.

5. A surgical device according to any of the preceding claims, **characterised in that** the distal end (52) is enlarged relative to the shaft (12, 48).

6. A surgical device according to any of the preceding claims, **characterised in that** the cap (20, 50) is arranged such that in use the cap (20, 50) contacts the surface of the tissue substantially circumferentially even when the shaft (12, 48) is inserted other than normally to the tissue surface.

7. A surgical device according to any of the preceding claims, **characterised in that** the cap (20, 50) has a substantially frusto conical shape with the shaft (12, 48) being attached to the proximal smaller end thereof.

8. A surgical device according to claim 7, **characterised in that** one or more openings (30) may be provided extending partway up the side walls of the cap (20, 50) from the distal larger end thereof.

9. A surgical device according to any of the preceding claims, **characterised in that** the engagement formations (18,46) are provided at a 120° or less spacing around the shaft (12, 48).

10. A surgical device according to any of the preceding claims, **characterised in that** the engagement formations (18,46) are provided in a plurality of groups of formations, with each group spaced longitudinally from each other on the shaft (12, 48).

11. A surgical device according to any of the preceding claims, **characterised in that** the engagement formations (18,46) are circumferentially equispaced.

12. A surgical device according to any of the preceding claims, **characterised in that** the engagement formations are in the form of barbs (18, 46).

13. A surgical device according to claim 12, **characterised in that** the barbs (18, 46) are rearwardly pointing.

14. A surgical device according any of the preceding claims, **characterised in that** engagement formations (18,46) are provided on or adjacent to the distal end (52) of the shaft (12, 48).

15. A surgical device according any of the preceding claims, **characterised in that** the shaft (12, 48) has a uniform cross-section along its length from the distal end (52).

16. A surgical device according any of the preceding claims, **characterised in that** the shaft (12, 48) cross-section is circular, elliptical or polygonal.

17. A surgical device according any of the preceding claims, **characterised in that** the shaft (12, 48) cross-section is triangular or square.

18. A surgical device according any of the preceding claims, **characterised in that** the shaft (12, 48) cross-section has a plurality of circumferential lobes (14).

19. A surgical device according to claim 18, **characterised in that** the lobes (14) are equispaced.

20. A surgical device according any of the preceding claims, **characterised in that** the cap (20, 50) comprises a formation (22) engageable with a surgical tool.

21. A surgical device according to claim 20, **characterised in that** the formation (22) is provided on the proximal end of the cap (20, 50).

22. A surgical device according to claim 20 or 21, **characterised in that** the cap (20, 50) comprises a recess (28) or projection (22).

23. A surgical device according to any of claims 20 to 22, **characterised in that** the formation (22) is shaped such as to permit the device to be used arthroscopically.

24. A surgical device according any of the preceding claims, **characterised in that** the device is made of a resorbable material.

25. A surgical device according to claim 24, **characterised in that** the resorbable material includes a resorbable polymer or copolymer.

26. A surgical device according to claim 25, **characterised in that** the polymer or copolymer is from the range of bioabsorbable polymers including β-hydroxybutyrate (hydroxybutanoate), hydroxyvalerate (hydroxypentanoate), lactic acid (2-hydroxypropanoic acid) or glycolic acid (2-hydroxyethanoic acid).

27. A surgical device according to any of claims 24 to 26, **characterised in that** the resorbable material is a composite.

28. A surgical device according to claim 27, **characterised in that** the composite comprises a particulate or fibrous solid material.

29. A surgical device according to claim 28, **characterised in that** the solid material comprises any of hydroxyapatite, tri-calcium phosphate, calcium carbonate, calcium sulphate, magnesium oxide or bioactive glass.

## Patentansprüche

1. Chirurgische Vorrichtung zum Schliessen von Körpergewebe, wobei die Vorrichtung aufweist: einen Schaft (12, 48), der in das Gewebe eingeführt werden kann; eine hohle Kappe (22, 40) am proximalen Ende des Schaftes (12, 48), die im wesentlichen mit der Oberfläche des Gewebes in Eingriff bringbar ist; und eine Vielzahl von Gewebe-Eingriffsformationen (18, 46) an dem Schaft (12, 48), um ein Herausziehen der Vorrichtung aus dem Gewebe zu verhindern, **dadurch gekennzeichnet, dass** die Kappe (20, 40) biegsam ist, so dass mit der Oberfläche des Gewebes ein guter Eingriff in Umfangsrichtung erzeugt wird.

2. Chirurgische Vorrichtung nach Anspruch 1, **dadurch gekennzeichnet, dass** das distale Ende des Schafts (12, 48) mit einer scharfen Spitze (16) versehen ist.

3. Chirurgische Vorrichtung nach Anspruch 1, **dadurch gekennzeichnet, dass** das distale Ende (52) des Schaftes (12, 48) stumpf ist.

4. Chirurgische Vorrichtung nach Anspruch 3, **dadurch gekennzeichnet, dass** das distale Ende (42) des Schafts (12, 48) abgerundet ist.

5. Chirurgische Vorrichtung nach einem der vorhergehenden Ansprüche, **dadurch gekennzeichnet, dass** das distale Ende (52) bezüglich des Schafts (12, 48) aufgeweitet ist.

6. Chirurgische Vorrichtung nach einem der vorhergehenden Ansprüche, **dadurch gekennzeichnet, dass** die Kappe (20, 50) so angeordnet ist, dass bei der Verwendung die Kappe die Oberfläche des Gewebes im wesentlichen entlang der Umfangsrichtung selbst dann berührt, wenn der Schaft (12, 48) anders als normal zur Gewebeoberfläche eingeführt wird.

7. Chirurgische Vorrichtung nach einem der vorhergehenden Ansprüche, **dadurch gekennzeichnet, dass** die Kappe (20, 50) im wesentlichen kegelstumpfförmig ist, wobei der Schaft (12, 48) an ihrem proximalen kleineren Ende befestigt ist.

8. Chirurgische Vorrichtung nach Anspruch 7, **dadurch gekennzeichnet, dass** eine oder mehrere Öffnungen (30) vorhanden sein können, die sich an den Seitenwänden der Kappe (20, 50) von ihrem distalen grösseren Ende aus teilweise nach oben erstrecken.

9. Chirurgische Vorrichtung nach einem der vorhergehenden Ansprüche, **dadurch gekennzeichnet, dass** die Eingriffsformationen (18, 46) mit einer Beabstandung von 120° oder weniger um den Schaft (12, 48) vorgesehen sind.

10. Chirurgische Vorrichtung nach einem der vorhergehenden Ansprüche, **dadurch gekennzeichnet, dass** die Eingriffsformationen (18, 46) in einer Vielzahl von Gruppen aus Formationen vorgesehen sind, wobei die Gruppen voneinander jeweils in Längsrichtung an dem Schaft (12, 48) beabstandet sind.

11. Chirurgische Vorrichtung nach einem der vorhergehenden Ansprüche, **dadurch gekennzeichnet, dass** die Eingriffsformationen (18, 46) in der Umfangsrichtung gleichmässig beabstandet sind.

12. Chirurgische Vorrichtung nach einem der vorhergehenden Ansprüche, **dadurch gekennzeichnet, dass** die Eingriffsformationen die Form von Widerhaken (18, 46) haben.

13. Chirurgische Vorrichtung nach Anspruch 12, **dadurch gekennzeichnet, dass** die Widerhaken (18, 46) in die Rückwärtsrichtung weisen.

14. Chirurgische Vorrichtung nach einem der vorhergehenden Ansprüche, **dadurch gekennzeichnet, dass** die Eingriffsformationen (18, 46) an oder neben dem distalen Ende (52) des Schaft (12, 48) vorgesehen sind.

15. Chirurgische Vorrichtung nach einem der vorhergehenden Ansprüche, **dadurch gekennzeichnet, dass** der Schaft (12, 48) entlang seiner Länge vom distalen Ende (52) aus einen gleichförmigen Querschnitt hat.

16. Chirurgische Vorrichtung nach einem der vorhergehenden Ansprüche, **dadurch gekennzeichnet, dass** der Querschnitt des Schafts (12, 48) kreisförmig, elliptisch oder polygonal ist.

17. Chirurgische Vorrichtung nach einem der vorhergehenden Ansprüche, **dadurch gekennzeichnet, dass** der Querschnitt des Schafts (12, 48) dreieckig oder quadratisch ist.

18. Chirurgische Vorrichtung nach einem der vorhergehenden Ansprüche, **dadurch gekennzeichnet, dass** der Querschnitt des Schafts (12, 48) eine Vielzahl von Ausbuchtungen (14) entlang der Umfangsrichtung hat.

19. Chirurgische Vorrichtung nach Anspruch 18, **dadurch gekennzeichnet, dass** die Ausbuchtungen (14) gleichmässig beabstandet sind.

20. Chirurgische Vorrichtung nach einem der vorhergehenden Ansprüche, **dadurch gekennzeichnet, dass** die Kappe (20, 50) eine Formation (22) aufweist, die mit einem chirurgischen Werkzeug in Eingriff bringbar ist.

21. Chirurgische Vorrichtung nach Anspruch 20, **dadurch gekennzeichnet, dass** die Formation (22) an dem proximalen Ende der Kappe (20, 50) vorgesehen ist.

22. Chirurgische Vorrichtung nach Anspruch 20 oder 21, **dadurch gekennzeichnet, dass** die Kappe (20, 50) eine Vertiefung (28) oder einen Vorsprung (22) aufweist.

23. Chirurgische Vorrichtung nach einem der Ansprüche 20 bis 22, **dadurch gekennzeichnet, dass** die Formation (22) so geformt ist, dass sie eine arthroskopische Verwendung der Vorrichtung gestattet.

24. Chirurgische Vorrichtung nach einem der vorhergehenden Ansprüche, **dadurch gekennzeichnet, dass** die Vorrichtung aus einem resorbierbaren Material besteht.

25. Vorrichtung nach Anspruch 24, **dadurch gekennzeichnet, dass** das resorbierbare Material ein resorbierbares Polymer oder Copolymer enthält.

26. Chirurgische Vorrichtung nach Anspruch 25, **dadurch gekennzeichnet, dass** das Polymer oder Copolymer aus der Gruppe bioabsorbierbarer Polymere stammt, welche Beta-Hydroxybutyrat (Hydroxybutanoat), Hydroxyvalerat (Hydroxypentanoat), Milchsäure (2-Hydroxypropansäure) oder Glykolsäure (2-Hydroxyethansäure) enthält.

27. Chirurgische Vorrichtung nach einem der Ansprüche 24 bis 26, **dadurch gekennzeichnet, dass** das resorbierbare Material ein Verbundmaterial ist.

28. Chirurgische Vorrichtung nach Anspruch 27, **dadurch gekennzeichnet, dass** das Verbundmaterial ein teilchenförmiges oder faserförmiges festes Material aufweist.

29. Chirurgische Vorrichtung nach Anspruch 28, **dadurch gekennzeichnet, dass** das feste Material Hydroxyapatit, TriCalciumphosphat, Calciumcarbonat, Calciumsulfat, Magnesiumoxid oder bioaktives Glas aufweist.

## Revendications

1. Dispositif chirurgical d'obturation de tissus corporels, comprenant une tige (12, 48) qui peut être insérée dans le tissu, un chapeau creux (20, 50) à l'extrémité proximale de la tige (12, 48) qui peut être engagé sensiblement contre la surface du tissu, et une pluralités de structures (18, 46) d'engagement du tissu sur la tige (12, 48) pour empêcher le dispositif d'être retiré du tissu, **caractérisé en ce que** le chapeau (20, 50) est flexible de sorte qu'un bon engagement circonférentiel avec la surface du tissu est réalisé.

2. Dispositif chirurgical selon la revendication 1 **caractérisé en ce que** l'extrémité distale de la tige (12, 48) est munie d'une pointe aiguisée (16).

3. Dispositif chirurgical selon la revendication 1 **caractérisé en ce que** l'extrémité distale (52) de la tige (12, 48) est émoussée.

4. Dispositif chirurgical selon la revendication 3 **caractérisé en ce que** l'extrémité distale (52) de la tige (12, 48) est arrondie.

5. Dispositif chirurgical selon l'une quelconque des revendications précédentes **caractérisé en ce que** l'extrémité distale (52) est agrandie par rapport à la tige (12, 48).

6. Dispositif chirurgical selon l'une quelconque des revendications précédentes **caractérisé en ce que** le chapeau (20, 50) est agencé de façon que, en utilisation, le chapeau (20, 50) soit en contact avec la surface du tissu sensiblement de manière circonférentielle même quand la tige (12, 48) est insérée autrement que normalement sur la surface du tissu.

7. Dispositif chirurgical selon l'une quelconque des revendications précédentes **caractérisé en ce que** le chapeau (20, 50) présente une forme sensiblement tronconique avec la tige (12, 48) fixé à l'extrémité proximale plus petite de celle-ci.

8. Dispositif chirurgical selon la revendication 7 **caractérisé en ce qu'**une ou plusieurs ouvertures (30) peuvent être ménagées en s'étendant jusqu'à mi-chemin des parois latérales du chapeau (20, 50) à partir de l'extrémité distale plus grande de celui-ci.

9. Dispositif chirurgical selon l'une quelconque des revendications précédentes **caractérisé en ce que** les structures d'engagement (18, 46) sont ménagées à des espacements de 120 ° ou moins autour de la tige (12, 48).

10. Dispositif chirurgical selon l'une quelconque des revendications précédentes **caractérisé en ce que** les structures d'engagement (18, 46) sont ménagées en une pluralité de groupes de structures, chaque groupe étant espacé longitudinalement de chaque autre sur la tige (12, 48).

11. Dispositif chirurgical selon l'une quelconque des revendications précédentes **caractérisé en ce que** les structures d'engagement (18, 46) sont équidistantes circonférentiellement.

12. Dispositif chirurgical selon l'une quelconque des revendications précédentes **caractérisé en ce que** les structures d'engagement ont la forme de barbes (18, 46).

13. Dispositif chirurgical selon la revendication 12 **caractérisé en ce que** les barbes (18, 46) pointent vers le bas.

14. Dispositif selon l'une quelconque des revendications précédentes **caractérisé en ce que** les structures d'engagement (18, 46) sont agencées sur ou adjacentes à l'extrémité distale (52) de la tige (12, 48).

15. Dispositif chirurgical selon l'une quelconque des revendications précédentes **caractérisé en ce que** la tige (12, 48) présente une section transversale uniforme suivant sa longueur à partir de l'extrémité distale (52).

16. Dispositif chirurgical selon l'une quelconque des revendications précédentes **caractérisé en ce que** la section transversale de la tige (12, 48) est circulaire, elliptique ou polygonale.

17. Dispositif chirurgical selon l'une quelconque des revendications précédentes **caractérisé en ce que** la section transversale de la tige (12, 48) est triangulaire ou carrée.

18. Dispositif chirurgical selon l'une quelconque des revendications précédentes **caractérisé en ce que** la section transversale de la tige (12, 48) possède une pluralité de lobes circonférentiels (14).

19. Dispositif chirurgical selon la revendication 18 **caractérisé en ce que** les lobes (14) sont équidistants.

20. Dispositif chirurgical selon l'une quelconque des revendications précédentes **caractérisé en ce que** le chapeau (20, 50) comporte une structure (22) pouvant coopérer avec un outil chirurgical.

21. Dispositif chirurgical selon la revendication 20 **caractérisé en ce que** la structure (22) est agencée sur l'extrémité proximale du chapeau (20, 50).

22. Dispositif chirurgical selon la revendication 20 ou 21 **caractérisé en ce que** le chapeau (20, 50) comporte un évidement (28) ou une saillie (22).

23. Dispositif chirurgical selon l'une quelconque des revendications 20 à 22 **caractérisé en ce que** la structure (22) est profilée de façon à permettre au dispositif d'être utilisé de manière arthroscopique.

24. Dispositif chirurgical selon l'une quelconque des revendications précédentes **caractérisé en ce que** ledit dispositif est réalisé en une matière résorbable.

25. Dispositif chirurgical selon la revendication 24 **caractérisé en ce que** le matériau résorbable comprend un polymère pu un copolymère résorbable.

26. Dispositif chirurgical selon la revendication 25 **caractérisé en ce que** le polymère ou le copolymère est de la gamme des polymères bioabsorbables comportant le β-hydroxybutyrate (hydroxybutanoate), l'hydroxyvalérate(hydroxypentanoate),l'acide lactique(acide 2-hydroxypropanoïque) ou l'acide glycolique (acide 2-hydroxyéthanoïque).

27. Dispositif chirurgical selon l'une quelconque des revendications 24 à 26 **caractérisé en ce que** le matériau résorbable est un composite.

28. Dispositif chirurgical selon la revendication 27 **caractérisé en ce que** le composite comporte un matériau solide particulaire ou fibreux.

29. Dispositif chirurgical selon la revendication 28 **caractérisé en ce que** le matériau solide comprend l'un quelconque de l'hydroxyapatite, du phosphate de tri-calcium, du carbonate de calcium, du sulfate de calcium, de l'oxyde de magnésium ou du verre bioactif.
